(19) Europäisches Patentamt
European Patent Office
Office européen des brevets

(11) Publication number: 0 186 223
A1

(12) EUROPEAN PATENT APPLICATION

(21) Application number: 85201838.1

(22) Date of filing: 11.11.85

(51) Int. Cl.4: C07D 209/52 , C07D 209/96

(30) Priority: 16.11.84 IT 2362384

(43) Date of publication of application: 02.07.86 Bulletin 86/27

(84) Designated Contracting States: AT BE CH DE FR GB LI LU NL SE

(71) Applicant: CONSIGLIO NAZIONALE DELLE RICERCHE
Viale Liegi 48/B
I-00198 Roma(IT)
Applicant: ENICHEM SINTESI S.p.A.
Via Ruggero Settimo 55
I-90139 Palermo(IT)

(72) Inventor: Chiusoli, Gian Paolo
Via Solferino 18/bis
I-43100 Parma(IT)
Inventor: Costa, Mirco
Piazza A. Fontanesi 11
I-42100 Reggio Emilia(IT)
Inventor: Gerbella, Marco
Via Bandi 4
I-43100 Parma(IT)
Inventor: Salerno, Giuseppe
Coop. "The Sun" c/ da Bosco Sottano
I-87100 Cosenza(IT)

(74) Representative: Roggero, Sergio et al
Ing. Barzanò & Zanardo Milano S.p.A. Via Borgonuovo 10
I-20121 Milano(IT)

# (54) 3-AZA-7-hydroxy-2,2,4,4-tetraalkylbicyclo[3.3.0] octonanes;process for their preparation, intermediates suitable to that purpose, and process for the production of these latter.

(57) Diacetylenic compounds belonging to the class of ditert-propargylamines are catalitically reacted with carbon monoxide and alkoxides or hydroxides of alkali metals, to yield carbonyl derivatives of formula:

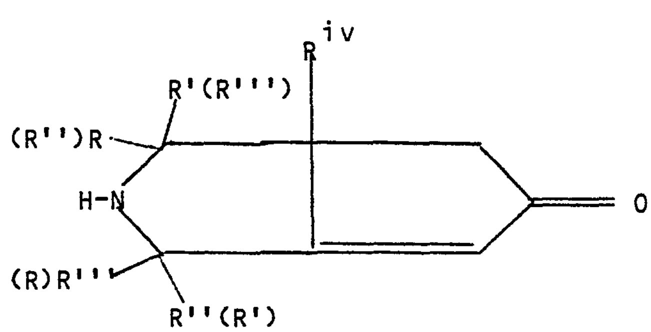

wherein R, R', R'', and R''', either equal to or different from each other, are alkyl radicals containing up to 12 carbon atoms, also bonded to each other, to form a ring; $R^{iv}$ is hydrogen or a straight alkoxy radical containing up to 4 carbon atoms.

The said carbonyl radicals are interesting intermediates for the preparation of antioxidizers for plastic materials; among these, particularly important are the diastereoisomer alcohols of formula:

EP 0 186 223 A1

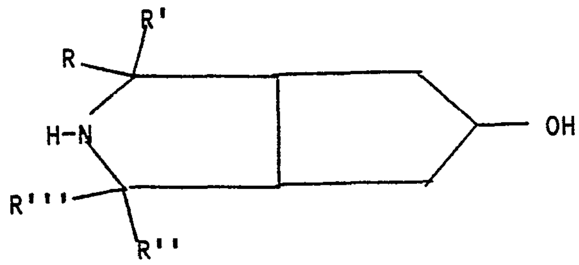

wherein the substituents have the above-reported meanings.

"3-AZA-7-HYDROXY-2,2,4,4-TETRAALKYLBICYCLO[3.3.0]OCTANES, PROCESS FOR THEIR PREPARATION, INTERMEDIATES SUITABLE TO THAT PURPOSE, AND PROCESS FOR THE PRODUCTION OF THESE LATTER".

The present invention relates to 3-aza-7-hydroxy-2,2,4,4-tetraalkylbicyclo[3.3.0]octanes, compounds useful as antioxidizers for polyolephins. It relates moreover to the carbonyl derivatives, used as the intermediates in the preparation of the said stabilizers.

It is known that the polyolephins, in particular polypropylene and polyethylene, undergo degradation with time, e.g., due to the exposure to the atmospheric agents, and to the consequent oxidation by the atmospheric oxygen.

Each degradation becomes evident as a loss of some physical characteristics of the polymers, such as e.g., a decrease in ultimate tensile strength, and a loss of flexibility. The decrease of the above indicated characteristics can be evaluated quantitatively by means of the measurement of the melt flow index of the polymers (MFI). An excessive increase in MFI denotes a severe breakdown of the polymer.

To the purpose of preventing such a breakdown, resort has been had to the addition of some amounts of stabilizer compounds to the polymers, of course caring that such compounds are not such as to alter the other characteristics of the polymers.

Polyolephinic compositions stabilized against the atmospheric agents are known. The U.S. Pat. N° 3,640,928 relates e.g. to the stabilization of synthetic polymers by means of compounds derivatives of pyperidine, whilst the U.S. Pat. N° 4,111,901 relates to derivatives of pyrrole as stabilizers of olephinic polymers against the U.V. radiations.

However, the presently known stabilizer agents, although they guarantee to the polymer the effects for which they are recommended, must sometimes be added to the same polymer in rather high amounts, or they are to be prepared according to techniques not always easily accomplishable.

We have now found, that is the object of the present invention, a new class of compounds showing outstanding characteristics as antioxidizers for the plastic materials, combined with such structural characteristics as to allow particularly satisfactory applicative characteristics to be achieved.

Such compounds are obtained by starting from carbonyl derivatives, which are the products corresponding to the condensation of a cyclopentenonic ring with a pyrrolidinic ring, being in their turn the second object of the present invention, through a series of reactions of easy and immediate accomplishment.

The compounds being the object of the present invention, useful as antioxidizers for polyolephins, are 3-aza-7-hydroxy-2,2,4,4-tetraalkylbicyclo[3.3.0]octanes of formula:

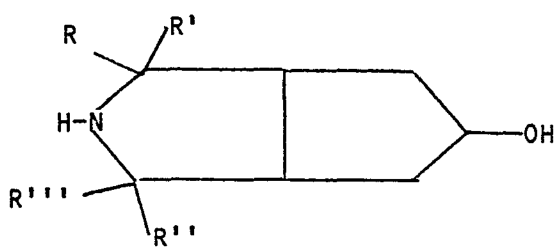

(I)

wherein R, R', R" and R"', either equal to or different to each other, are alkyl radicals containing up to 12 carbon atoms, also bonded to each other to form a ring.

The said compounds are obtained by means of the hydrogenation, in particular on Raney nickel, of carbonyl derivatives, which are products corresponding to the condensation of a cyclopentenonic ring with a sterically hindered pyrrolidinic ring, and having the following general formula:

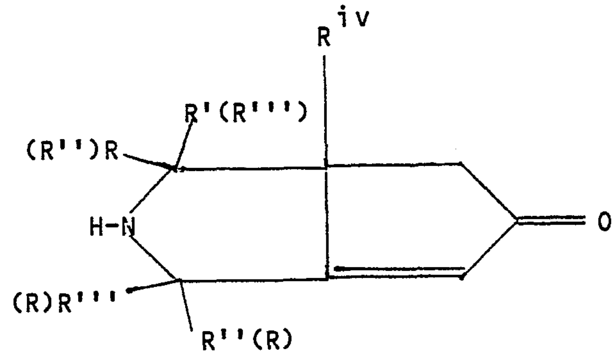

(II)

wherein R, R', R'', and R''' have the above-reported meaning, and $R^{iv}$ may be hydrogen or an alkoxy radical containing up to 4 carbon atoms.

Such carbonyl derivatives are obtained by means of a process, which is a third object of the present invention, consisting in reacting acetylenic compounds of formula:

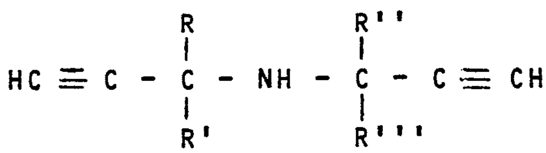

(III)

with carbon monoxide and a hydroxide or an alkoxide of an alkaline metal of formula $R^{v}OM$, wherein $R^{v}$ represents H or a straight alkyl of up to 4 carbon atoms, and M represents the alkali metals, such a reaction occurring in the presence of a catalyst constituted by palladium as a complex of $Pd^{o}$ with labile olephinic ligands, or as finely divided metal outcoming from the reduction of palladium salts or complexes with or without an inert material support.

In an alternative embodiment, instead of the alkoxide, hydroxide + alcohol may be used.

The reaction is carried out under extremely mild conditions, simply by dissolving the diacetylenic amine and the alkoxide or the hydroxide in a suitable solvent, in amine : alkoxide or hydroxide molar ratios of from 0.1 : 1 to 1 : 0.1, in the presence of a catalyst as defined above, under stirring at room temperature (10-50°C), under a carbon monoxide atmosphere. The absorption of this latter can be easily monitored, and the reaction is considered as ended when the absorption ceases; at that time, the separation of the products can be carried out, according to conventional techniques. In particular, it can be filtered off the catalyst, which can be used again for further cycles, and thereafter the solvent and then the product can be distilled off. The product is generally separated at a good purity level.

As briefly outlined, the reaction is carried out in the presence of a solvent. In case $R^{iv}$ is hydrogen, such a solvent is a mixture of water with a compound miscible with it and with the hydroxide of the alkali metal; preferably, mixtures of aliphatic ethers and alicyclic ethers with water containing about 10% by volume of water are used.

In case $R^{iv}$ is 0-alkyl, the solvent may be the alcohol corresponding to the desired alkoxy group, or another solvent inert under the reaction conditions, such as the aforementioned ethers.

The so-obtained carbonyl compounds must be submitted then to hydrogenation for the preparation of the alcohols, antioxidizers for plastic materials, being the main object of the present invention.

Such a hydrogenation, which comprises also the hydrogenolysis of the alkoxy group, is preferably carried out by using hydrogen over Raney nickel.

The invention shall be illustrated now by means of the following Examples, by which however it should not be considered as being limited.

Example 1

Into a flask with side vacuum nozzle there are introduced, under inert atmosphere, 0.209 g (about 0.2 mmol) of Pd/C at 10% of metal, 0.461 g (about 3.093 mmol) of III, with R, R', R'', R''' = methyl (IIIa), 0.75 ml of methanolic solution of MeONa (about 0.75 mmol, concentration 1.0 mol/l), and 9.25 ml of MeOH. The flask is then connected to a CO-containing burette, and is dipped into a bath thermo-regulated at 25°C. After about 72 hours, the CO absorption being finished, the reaction is interrupted, the reaction mixture is centrifuged and washed with MeOH to the purpose of recovering the catalyst, which is used again for subsequent carbonylations. The calibration by gas-chromatographic procedure is carried out (using methyl laurate as the internal standard). The following results are obtained:

IIa, $R^{iv}$ = OMe 0.577 g, corresponding to 89% of fed IIIa, equal to about 14 mol/mol of Pd.

IIIa, 0.09 g, corresponding to 2% of fed IIIa.

IIa, $R^{iv}$ = H 0.024 g, corresponding to 4% of fed IIIa, and a small amount of heavier product.

Similar results are obtained by using EtONa and nBuONa as the alkoxides.

With EtONa, by starting from 0.310 g of Pd/C at 10% (0.293 mmol), 0.478 (3.207 mmol) of IIIa, 3.0 ml of ethanolic solution of EtONa (about 3.0 mmol, concentration 1.0 mol/l) and 7.0 ml of EtOH, the following results are obtained:

IIa, $R^{iv}$ = OEt 0.624 g, corresponding to 87.3% of fed IIIa, equivalent to 9.6 mol/mol of Pd.

IIIa 0.006 g, corresponding to 1% of fed IIIa

IIa , $R^{iv}$ = H 0.040 g, corresponding to 6% of fed IIIa, and a small amount of a heavier product.

With n-BuONa, by starting from 0.314 g of Pd/C at 10% (0.297 mmol), 0.452 g of IIIa (3.031 mmol), 3.0 ml (3.057 mmol) of butanolic solution of nBuONa (as 1.013 mol/l), and 7 ml of BuOH, the following results are obtained:

IIa , $R^{iv}$ = nOBu 0.722 g, corresponding to 95% of fed IIIa, equal to 9.5 mol/mol of Pd.

111a 0.15 g, corresponding to 3% of fed IIIa.

IIa , $R^{iv}$ = H 0.005 g, corresponding to 1% of fed IIIa.

Characterization of Product II: R, R', R'', R''' = methyl (IIa)

IIa $R^{iv}$ = OMe Colourless solid, m.p. = 55 - 57°C Mass spectrum m/e 209 ($M^+$), 194, 178, 162, 152, 137, 120, 109, 58 IR film ($cm^{-1}$) 3330, 2980, 1720, 1640, 1465, 1380, 1365, 1165, 1080, 860, 815, 740.

UV ethanol 95° λmax 225 nm ε = 6000

1HNMR (200 MHz, $CDCl_3$, TMS, ε) 6.16 C=$C^{/H}$, 3.12 (s)$OCH_3$, 2.29-2.53 (AB system), $CH_2$, 1.53 (s)$CH_3$, 1.52 (s)$CH_3$, 1.39 (s)CH, 1.19 (s)$CH_3$.

$^{13}$C NMR ($CDCl_3$, TMS ppm) 206.6 (C=0), 188.6 (>C=) 126.9 (=$C^{/H}$), 92.2 (≡C-0), 61.6 (> $CMe_2$), 56.7 (>$CMe_2$), 49.8 (OMe), 39.9 ($CH_2$), 30.7 ($CH_3$), 28.5 ($CH_3$), 26.3 ($CH_3$), 21.9 ($CH_3$).

IIa$R^{iv}$ = OEt Colourless solid, m.p. = 39 41°C Mass spectrum m/e 223 ($M^+$), 208, 194, 178, 166, 148, 138, 120, 110, 109, 95, 67, 58 IR KBr ($cm^{-1}$) 3300, 2980, 1720, 1635, 1465, 1375, 1175, 1080, 860, 815.

UV ethanol 95° λmax 224 nm ε = 6000.

IIa $R^{iv}$ = n-OBu colourless oil mass spectrum m/e 251 ($M^+$), 236, 194, 178, 163, 162, 148, 138, 120, 110, 95, 67, 58, 57.

IR film ($cm^{-1}$) 3310, 2980, 1715, 1635, 1460, 1380, 1175, 1080, 860, 815.

UV ethanol 95° λmax 231 nm ε = 7100.

Example 2

Into a flask provided with side vacuum hose, there are introduced, under an inert atmosphere, 0.309 g (about 0.291 mmol) of Pd/C at 10% of metal, 0.571 g (3.833 mmol) of IIIa, 0.040 g (1.0 mmol) of solid NaOH and 10 ml of MeOH. The process is carried out as in Example 1. After 72 hours, at the end of CO absorption, the reaction is interrupted. The calibration, as carried out by gas-chromatographic route, has given the following results:

IIa $R^{iv}$ = OMe 0.714 g corresponding to 89% of fed IIIa, equal to 12 mol/mol of Pd.

IIIa 0.012 g corresponding to 2% of fed IIIa

IIa, $R^{iv}$ = H 0.028 g corresponding to 4% of fed IIIa, and a small amount of a heavier product.

The use of NaOH in EtOH and BuOH, even if maintains a high conversion, yields a decreasing selectivity to the product IIa, $R^{iv}$ = OEt and $R^{iv}$ = OBu.

Example 3

Into a flask provided with side vacuum nozzle, there are introduced under an inert atmosphere, 0.311 g (about 0.294 mmol) of Pd/C at 10% of metal, 0.491 g (about 3.295 mmol) of IIIa, 1.5 ml (1.5 mmol , concentration 1 mol/l) of methanolic solution of MeONa and 10 ml of MeOH.

The flask is connected to a CO-containing burette, and is dipped into an oil bath at 50°C. After 18 hours, at the end of CO absorption, the reaction is stopped and the separation of the reaction product is carried out as in Example 1. The calibration by the gas-chromatographic route has given the following results:

IIa, $R^{iv}$ = OMe 0.566 g corresponding to 82% of fed IIIa, equal to 9.2 mol/mol of Pd.

IIIa 0.015 g corresponding to 3% of fed IIIa.

IIa $R^{iv}$ = H 0.030 g corresponding to 5% of fed IIIa and small amounts of heavier products.

Example 4

Into a flask with side vacuum nozzle there are introduced, under inert atmosphere, 0.051 g (about 0.289 mmol) of $PdCl_2$, 0.519 g (about 3.48 mmol) of IIIa, 1.5 ml (about 1.5 mmol) of MeOna solution (concentration 1 mol/l) and 8.5 ml of MeOH. The reaction is carried out as in Example 1. After 96 hours at 25°C under stirring, after the end of CO absorption, the precipitated black Pd is filtered off, and the calibration by gas-chromatographic route is carried out. The following results are obtained:

IIa, $R^{iv}$ = OMe 0.305 g corresponding to 42% of fed IIIa, equal to 5 mol/mol of Pd.

IIIa 0.015 g corresponding to 3% of fed IIIa.

IIa $R^{iv}$ = H 0.025 g corresponding to 4% of fed IIIa and small amounts of heavier products.

Similar results are obtained by using a $Pd^{o}$ complex, and namely $Pd_2(DBA)_3CHC_3$, wherein DBA = dibenzylideneacet one.

Example 5

Into a flask provided with side vacuum nozzle there are introduced, under inert atmosphere, 0.310 g (0.292 mmol) of Pd/C at 10% of metal, 0.488 g (about 3.276 mmol) of IIIa, 0.344 g (8,61 mmol) of NaOH dissolved in 1 ml of $H_2O$ and 9 ml of dioxane. The flask is connected to a CO-containing burette, and is dipped into an oil bath thermo-regulated at 40°C. After about 70 hours, after the end of the CO absorption, the reaction is interrupetd, and the separation of the reaction products is carried out with the already described methods. The calibration is carried out by gas-chromatographic route, using n-hexadecane as the internal standard. The following results are obtained.

IIIa conversion: 98%

IIa, $R^{iv}$ = H

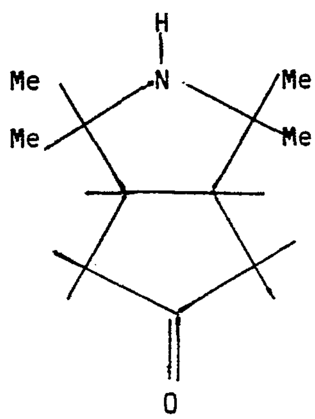

0.282 g corresponding to 40% of fed IIIa.

0.020 g corresponding to 3% of fed IIIa.

and small percentages of other gas-chromatographable products.

Similar results are obtained by using THF (tetrahydrofuran) and dimethoxyethane as the solvents. Characterization of the product IIa ($R^{iv}$ = H):

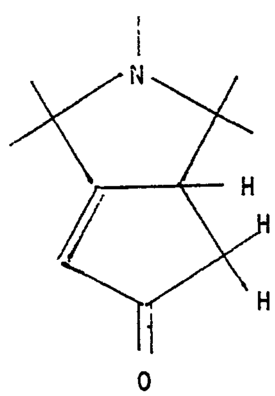

White solid, m.p. 37 ÷ 38°C

Mass spectrum: m/e 179 ($M^+$)

164, 147, 136, 122, 107, 94, 93, 79, 77, 58.

IR film ($cm^{-1}$) 3310, 2980, 1710, 1630, 1460, 1380, 1365, 1250, 1165, 970, 930, 845, 820, 700.

UV ethanol 95°

λmax 233 nm ε = 6440

Example 6

Hydrogenation of II with Raney Ni

Into a flask with side vacuum nozzle, there are introduced under inert atmosphere 0.5 ml of Raney-Ni suspension in MeOH, 0.667 g of purified compound IIa (3.19 mmol), and 17 ml of MeOH. Nitrogen is evacuated, and the reaction flask is connected to a hydrogen-containing burette.

After the end of $H_2$ absorption, Raney Ni is filtered off, washing with MeOH. The alcohol is distilled off under reduced pressure, and a white solid is obtained. This solid is purified by washing it with ethyl acetate. 0.500 g are recovered of solid product constituted by diastereo-isomers, one of which is predominant.

M. p. 199-200°C.

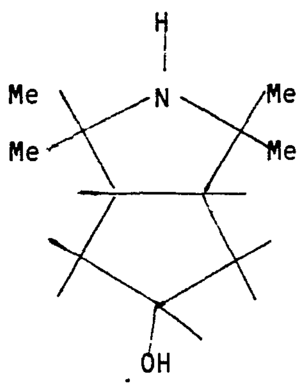

Mass m/e 183 ($M^+$), 182, 168, 150, 133, 122, 109, 99, 93, 84, 67, 58, 57.

IR KBr pellet ($cm^{-1}$) 3260, 3090, 2970, 1480, 1340, 1200, 1080, 850.

Example 7

By operating as in Example 1, the compound IIIb

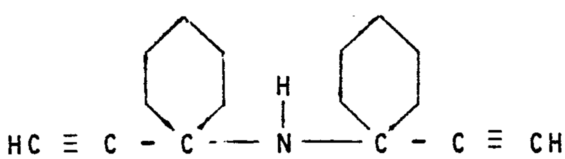

IIIb

is reacted with the same reactants, and under the same conditions.

By using 0.352 g (about 1.537 mmol) of IIIb, 0.154 g (about 0.145 mmol) of Pd/C with 10% of metal, 0.75 ml of methanolic solution of MeONa (0.75 mmol, concentration 1.0 mol/l) and 9.25 ml of MeOH, the corresponding product IIb with $R^{iv}$ = OMe is obtained with high selectivity, and namely:

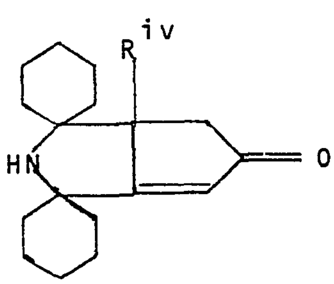

IIb 0.182 g corresponding to 41% of fed IIIb

IIIb 0.190 g corresponding to 54% of fed IIIb.

The compound IIb hydrogenated on Raney Ni yields the corresponding alcohol Ib.

## Claims

1. 3-Aza-7-hydroxy-2,2,4,4-tetraalkylbicyclo[3.3.0]-octanes of formula:

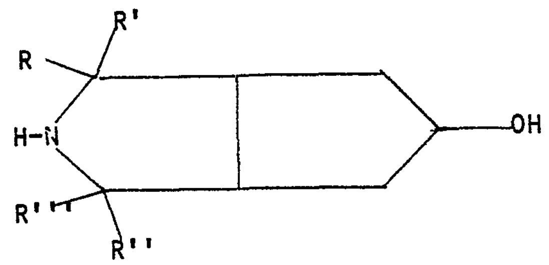

wherein R, R', R" and R"', either equal to or different from each other, are alkyl radicals containing up to 12 carbon atoms, also bonded to each other to form a ring.

2. Derivatives corresponding to the condensation of a cyclopentenonic ring and a pyrrolidinic ring of formula:

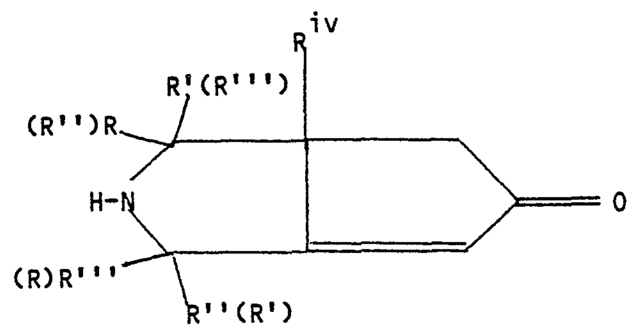

wherein R, R', R" and R"' have the meaning as reported in the preceding claim and $R^{iv}$ may be either hydrogen or an alkoxy radical containing up to 4 carbon atoms.

3. Process for the preparation of the compounds according to claim 2, consisting in reacting, in a suitable solvent, acetylenic compounds of formula:

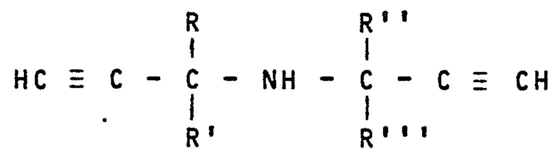

with carbon monoxide and a hydroxide or an alcoholate of an alkali metal of formula $R^{V}OM$, wherein $R^{V}$ represents hydrogen or a straight alkyl radical containing up to 4 carbon atoms, and M represents the alkali metal, in the presence of a palladium-based catalyst.

4. Process according to the preceding claim, characterized in that, if $R^{V}$ represents hydrogen, the reaction is carried out in the presence of a solvent constituted by a mixture of water and an ether selected among the aliphatic and alicyclic ethers, with water being present up to 10% by volume.

5. Process according to claim 3, characterized in that if $R^{V}$ represents an alkyl radical, the reaction is carried out in a solvent selected among the alcohol corresponding to the desired alkoxy group or aliphatic or alicyclic ethers.

6. Process according to claim 3, characterized in that the catalyst is selected among the complexes of $Pd^{o}$olephinic ligands and metal palladium in a finely divided form.

7. Process according to claim 3, characterized in that the reaction is carried out starting from an amine/hydroxy or alkoxy ratio comprised within the range of from 0.1 : 1 to 1 : 0.1.

8. Process according to claim 3, characterized in that the reaction is carried out at a temperature comprised within the range of from 10 to 50°C.

9. Process for the preparation of the compounds as of claim 1, consisting in submitting to hydrogenation the derivatives

as defined in claim 2.

European Patent Office

# EUROPEAN SEARCH REPORT

Application number

EP 85 20 1838

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | THE JOURNAL OF ORGANIC CHEMISTRY, vol. 30, no. 8, August 1965, pages 2645-2650; G.F. HENNION et al.: "Sterically crowded amines. IV. Secondary and tertiary bispropargylic amines and their hydrogenation products" * Page 2646; page 2647 "experimental" * | 3-8 | C 07 D 209/52<br>C 07 D 209/96 |
| X | GB-A-1 468 046 (I.C.I.) * Complete specification * | 3-8 | |
| X | CHEMICAL ABSTRACTS, vol. 100, 1984, page 435, abstract no. 34363u, Columbus, Ohio, US; G.P. CHIUSOLI et al.: "Diyne ring closure induced by palladium-catalyzed carbonylation" & J. ORGANOMET. CHEM. 1983, 255(3) * Abstract * | 3-8 | TECHNICAL FIELDS SEARCHED (Int. Cl.4)<br>C 07 D 209/00 |
| A | US-A-4 393 218 (COSTANZI et al.) * Abstract * | 1-9 | |
| X | FR-A-2 476 105 (ANIC SpA) * Claims * | 3-8 | |
| | --- -/- | | |

The present search report has been drawn up for all claims

| Place of search | Date of completion of the search | Examiner |
|---|---|---|
| THE HAGUE | 26-03-1986 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

European Patent Office

# EUROPEAN SEARCH REPORT

Application number

EP 85 20 1838



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 476 106 (ANIC SpA)<br>* Claims *<br>--- | 1-9 | |
| A | EP-A-0 093 467 (ANIC Spa)<br>* Abstract *<br>----- | 1-9 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 26-03-1986 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document